(19) Europäisches Patentamt
European Patent Office
Office européen des brevets

(11) Numéro de publication: **0 366 561 A2**

(12) # DEMANDE DE BREVET EUROPEEN

(21) Numéro de dépôt: 89402973.5

(22) Date de dépôt: 27.10.89

(51) Int. Cl.5: C07D 453/02 , C07D 487/08 , A61K 31/435 , A61K 31/40 , //(C07D487/08,209:00,209:00)

(30) Priorité: 28.10.88 IT 2245288

(43) Date de publication de la demande: 02.05.90 Bulletin 90/18

(84) Etats contractants désignés: AT BE CH DE ES FR GB GR IT LI LU NL SE

(71) Demandeur: ROUSSEL-UCLAF
35, boulevard des Invalides
F-75007 Paris(FR)

(72) Inventeur: Galliani, Giulio
13, Via Silva
I-Monza (MI)(IT)
Inventeur: Barzaghi, Fernando
Via Monteverdi, 21
I-20052 Monza Milan(IT)
Inventeur: Bonetti, Carla
Via Beccalino
Fontanella Bergamo(IT)
Inventeur: Toja, Emilio
Via Plezzo, 80
I-Milano(IT)

(74) Mandataire: Tonnellier, Marie-José et al
111, route de Noisy B.P. no 9
F-93230 Romainville(FR)

(54) **Nouveaux dérivés 1-azabicycloalkanes, leur procédé de préparation et leur application comme médicaments.**

(57) L'invention concerne les nouveaux dérivés 1-azabicyclo alkanes de formule (I) :

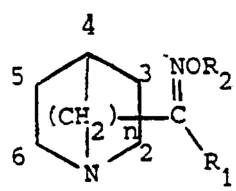

(I)

dans laquelle n représente 1, 2 ou 3, $R_1$ et $R_2$ identiques ou différents, représentent un hydrogène, un radical alkyle, alcényle ou alcynyle ($C_{1-6}$) éventuellement substitué par un hydroxy, un radical alkoxy ($C_{1-6}$), aralkyle (→ $C_{1-14}$), -(COOalc$_1$, -CON(alc)$_2$, alc$_1$ et alc$_2$ étant un alkyle ($C_{1-6}$), le radical -C($R_1$)-=NOR$_2$ étant en position 2, 3 ou 4, sous toutes les formes isomères et leurs mélanges, ainsi que les sels, leur procédé de préparation et leur application comme médicaments cholinomimétiques.

EP 0 366 561 A2

## Nouveaux dérivés 1-azabicycloalkanes, leur procédé de préparation et leur application comme médicaments.

La présente invention concerne de nouveaux dérivés 1-azabicycloalkanes, leur procédé de préparation et leur application comme médicaments.

L'invention a pour objet les composés de formule générale (I) :

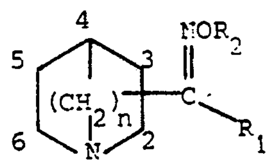

(I)

dans laquelle

- n représente le nombre 1, 2 ou 3,
- $R_1$ et $R_2$, identiques ou différents, représentent un atome d'hydrogène, un radical alkyle linéaire ramifié ou cyclique, un radical alcényle ou un radical alcynyle pouvant renfermer jusqu'à 6 atomes de carbone, $R_1$ et $R_2$ pouvant éventuellement être substitués par un radical hydroxy, un radical alkoxy renfermant jusqu'à 6 atomes de carbone, un radical aralkyle renfermant jusqu'à 14 atomes de carbone, un radical COOalc$_1$ dans lequel alc$_1$ représente un radical alkyle renfermant jusqu'à 6 atomes de carbone, un radical -CON(alc$_2$)$_2$ dans lequel alc$_2$ représente un radical alkyle renfermant jusqu'à 6 atomes de carbone, le radical -C($R_1$)-=NO$R_2$ étant en position 2, 3 ou 4, sous toutes leurs formes isomères possibles et leurs mélanges, ainsi que leurs sels d'addition avec les acides organiques ou minéraux.

Parmi les sels d'addition avec les acides, on peut citer ceux formés avec les acides minéraux, tels que les acides chlorhydrique, bromhydrique, sulfurique ou phosphorique ou avec les acides organiques comme l'acide formique, acétique, propionique, benzoïque, maléïque, fumarique, succinique, tartrique, citrique, oxalique, glyoxylique, aspartique, alcanesulfoniques, tels que les acides méthane ou éthanesulfoniques, arènesulfoniques tels que les acides benzène ou para-toluènesulfoniques.

Lorsque $R_1$ ou $R_2$ représente un radical alkyle saturé, linéaire ou ramifié, il s'agit de préférence d'un radical méthyle, éthyle, n-propyle, isopropyle, n-butyle, sec-butyle, isobutyle, n-pentyle, n-hexyle, tert-butyle, tert-pentyle, néopentyle ou n-hexyle.

Lorsque $R_1$ ou $R_2$ représente un radical alkyle cyclique, il s'agit de préférence d'un radical cyclopropyle, cyclobutyle, cyclopentyle, cyclohexyle, cyclopropylméthyle, cyclobutylméthyle, cyclopentylméthyle ou cyclohexylméthyle.

Lorsque $R_1$ ou $R_2$ représente un radical alcényle ou alcynyle, il s'agit de préférence d'un radical éthylénique comme, par exemple, un radical vinyle, allyle, 1,1-diméthylallyle, 2-butényle ou d'un radical acétylénique comme, par exemple, le radical éthynyle ou propynyle.

Par radical alkyloxy, on entend de préférence un radical méthoxy ou éthoxy, propoxy ou butoxy linéaire ou ramifié.

Le radical aralkyle est de préférence un radical benzyle ou phénéthyle.

Les radicaux alc$_1$ et alc$_2$ représentent de préférence les radicaux méthyle ou éthyle.

Parmi les composés préférés de l'invention, on peut citer les composés dans lesquels $R_2$ représente un radical alkyle, alcényle ou alcynyle renfermant jusqu'à 6 atomes de carbone ainsi que leurs sels d'addition avec les acides organiques ou minéraux.

L'invention a plus particulièrement pour objet les composés dans lesquels le radical -C($R_1$)=NO$R_2$ est en position 3, ainsi que leurs sels d'addition avec les acides organiques ou minéraux.

L'invention a notamment pour objet les composés dans lesquels n représente le nombre 1 et ceux dans lesquels n représente le nombre 2, ainsi que leurs sels d'addition avec les acides organiques ou minéraux.

Parmi les composés préférés de l'invention, on peut citer tout spécialement les composés dans lesquels $R_1$ représente un atome d'hydrogène ainsi que leurs sels d'addition avec les acides organiques ou minéraux, les composés dans lesquels lorsque n = 2, $R_1$ et $R_2$ ne représentent pas chacun un atome d'hydrogène, ceux dans lesquels $R_2$ représente un radical alkyle linéaire, ramifié ou cyclique renfermant jusqu'à 4 atomes de carbone comme par exemple un radical méthyle, ainsi que leurs sels d'addition avec les acides organiques ou minéraux.

L'invention a tout spécialement pour objet :

- le O-méthyloxime de 1-azabicyclo [2,2,2] octan-3-carboxaldéhyde ainsi que ses sels d'addition avec les

acides organiques ou minéraux,
- le O-méthyloxime de 1-azabicyclo [2,2,2] octan-3-yl éthanone ainsi que ses sels d'addition avec les acides organiques ou minéraux et particulièrement son chlorhydrate
- le O-méthyloxime de 1-aza-bicyclo [2,2,1] heptan-3-carboxaldéhyde ainsi que ses sels d'addition avec les acides organiques ou minéraux et particulièrement son sesqui oxalate.

Les composés de l'invention ont une activité cholinomimétique centrale très marquée et de longue durée d'action.

L'invention a donc pour objet les composés de formule (I) et leurs sels d'addition avec les acides organiques ou minéraux pharmaceutiquement acceptables, en tant que médicaments utiles notamment dans le traitement de la maladie d'Alzheimer, de la démence sénile et également dans le traitement des troubles de la mémoire.

L'invention a plus particulièrement pour objet en tant que médicaments, le composé de l'exemple 1 et celui de l'exemple 4.

La posologie usuelle est variable selon l'affection en cause, le sujet traité et la voie d'administration, elle peut être comprise entre 1 mg et 100 mg/jour, par exemple entre 1 et 15 mg/jour en une ou plusieurs prises pour le produit de l'exemple 1 administré par voie orale.

La présente invention a également pour objet les compositions pharmaceutiques renfermant comme principe actif au moins un produit de formule (I) ou l'un de ses sels avec les acides organiques ou minéraux pharmaceutiquement acceptables. Les compositions pharmaceutiques de l'invention peuvent être solides ou liquides et se présenter sous les formes pharmaceutiques couramment utilisées en médecine humaine, comme par exemple les comprimés simples ou dragéifiés, les gélules, les granulés, les suppositoires, les préparations injectables ; elles sont préparées selon les méthodes usuelles.

Le ou les principes actifs peuvent y être incorporés à des excipients habituellement employés dans ces compositions pharmaceutiques, tels que le talc, la gomme arabique, le lactose, l'amidon, le stéarate de magnésium, le beurre de cacao, les véhicules aqueux ou non, les corps gras d'origine animale ou végétale, les dérivés paraffiniques, les glycols, les divers agents mouillants, dispersants ou émulsifiants, les conservateurs.

L'invention a également pour objet un procédé de préparation, caractérisé en ce que l'on soumet un composé de formule générale (II) :

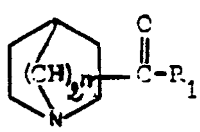

dans laquelle n et $R_1$ conservent la même signification que précédemment, à l'action d'un composé de formule générale (III) :

$R_2ONH_2$ (III)

dans laquelle $R_2$ conserve la même signification que précédemment, pour obtenir le composé de formule (I) correspondant, que l'on salifie le cas échéant.

Dans un mode de réalisation préféré du procédé de l'invention, les composés de formule (III) sont utilisés sous forme de chlorhydrate.

Les composés de formule (II) dans laquelle $R_1$ représente un atome d'hydrogène ou un radical alkyle sont connus d'une façon générale, voir à ce sujet Zhur Obshchei Khim 27, 72 (1957), Helv. Chim. Acta. 46, 2658 (1963), Helv. Chim. Acta 55, 2439 (1972), Heterocycles 24, 971 (1986), Helv. Chim. Acta 57, 2332 (1974).

Les composés de formule (II) dans lesquels $R_1$ représente un atome d'hydrogène peuvent être préparés selon le schéma réactionnel suivant :

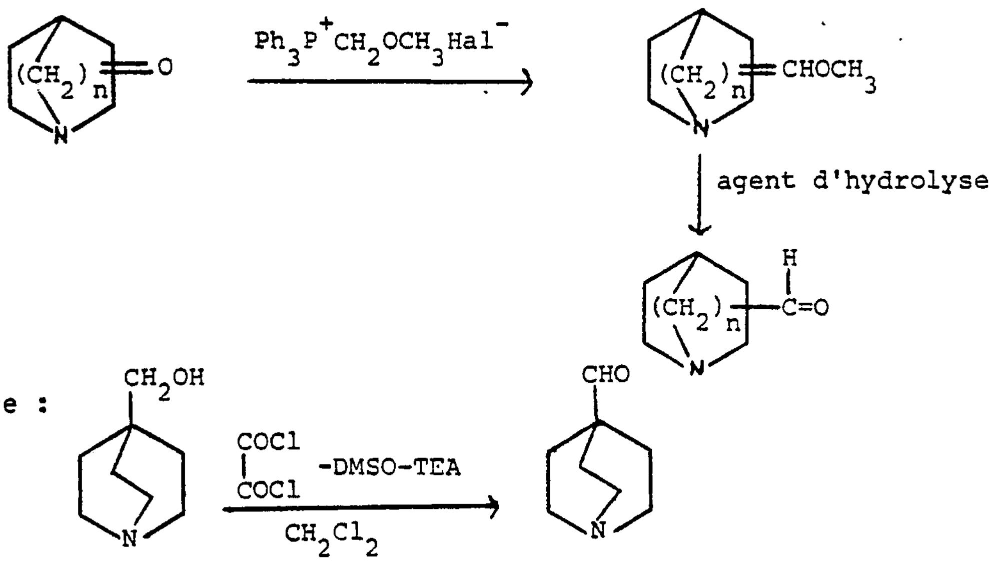


ou encore :

- Hal représente de préférence un atome de chlore ou de brome.
- L'agent d'hydrolyse est de préférence l'acide perchlorique.

Les exemples suivants illustrent l'invention sans toutefois la limiter.

**Exemple 1 : O-méthyloxime de 1-azabicyclo [2,2,2] octan-3-carboxaldéhyde.**

Dans 4,6 g de 3-méthoxy méthylidène quinuclidine (Heterocycles 244, 4-971 (1986)) en solution dans 46 cm3 de chloroforme, on ajoute à température ambiante 11 cm3 d'une solution aqueuse d'acide perchlorique à 70%. On ajoute, après 1 heure de contact, 2,5 g de chlorhydrate de méthoxylamine dans 10 cm3 d'eau et 10 cm3 de méthanol et maintient ainsi le milieu réactionnel pendant 1 nuit. On concentre, alcalinise à l'aide de soude 2N, extrait à l'acétate d'éthyle, évapore les solvants, reprend dans l'éther éthylique et filtre. On distille le filtrat à 150¤C sous 0,08 mbar et obtient 3,2 g de produit attendu.

| Analyse : $C_9H_{16}N_2O$ | | | |
|---|---|---|---|
| Calculé :<br>Trouvé : | C% 64,25<br>64,15 | H% 9,59<br>9,54 | N% 16,65<br>16,48 |

**Exemple 2 : O-éthyloxime de 1-azabicyclo [2,2,2] octan-3-carboxaldéhyde.**

Dans 2 g de 3-méthoxy méthylidène quinuclidine en solution dans 20 cm3 de chloroforme, on ajoute à température ambiante 4,8 cm3 d'une solution aqueuse d'acide perchlorique à 70%. On agite 1 heure, ajoute 1,4 g de chlorhydrate de O-éthylhydroxylamine dans 2,5 cm3 de méthanol et 2,5 cm3 d'eau, et maintient ainsi le milieu réactionnel pendant 1 nuit. On concentre, alcalinise à l'aide de soude, extrait à l'acétate d'éthyle, distille à 135¤C sous 0,05 mbar et obtient 2,05 g de produit attendu.

| Analyse : $C_{10}H_{18}N_2O$ | | | |
|---|---|---|---|
| Calculé :<br>Trouvé : | C% 65,90<br>65,89 | H% 9,86<br>9,78 | N% 15,37<br>15,23 |

**Exemple 3 : O-2-propynyloxime de 1-azabicyclo [2,2,2] octan-3-carboxaldéhyde.**

On opère comme à l'exemple 1 en utilisant au départ 1,75 g de 3-méthoxy méthylidène quinuclidine, 3,8 cm3 d'acide perchlorique et 1,22 g de chlorhydrate de O-2-propynylhydroxylamine (Brevet US 3,398,180 (1968)). Après avoir évaporé l'éther éthylique, on chromatographie le résidu sur silice (éluant : chloroforme-méthanol 7-3) puis distille à 190¤C sous 0,05 mbar et obtient 1,35 g de produit attendu.

| Analyse : $C_{11}H_{16}N_2O$ | | | |
|---|---|---|---|
| Calculé : | C% 68,72 | H% 8,39 | N% 14,57 |
| Trouvé : | 68,35 | 8,40 | 14,49 |

**Exemple 4 : Chlorhydrate de O-méthyloxime de (1-azabicyclo [2,2,2] octan-3-yl) éthanone.**

On chauffe 20 minutes au reflux 2,3 g de chlorhydrate de 3-acétyl quinuclidine (Helv. Chim. Acta (1963) 229, 2658) et 1,02 g de chlorhydrate de O-méthylhydroxylamine dans 30 cm3 de méthanol. On évapore le solvant, chromatographie le résidu sur silice (éluant : chloroforme-méthanol 7-3). Après cristallisation dans l'éthanol, dans l'éther, on obtient 1,8 g de produit attendu. F = 186-188¤C.

| Analyse : $C_{10}H_{18}N_2O$, HCl | | | |
|---|---|---|---|
| Calculé : | C% 53,80 | H% 8,80 | N% 12,55 |
| Trouvé : | 53,59 | 8,94 | 12,50 |

**Exemple 5 : O-2-propynyloxime de (1-azabicyclo [2,2,2] octane-3-yl) éthanone.**

On chauffe 20 minutes au reflux 1,8 g de chlorhydrate de 3-acétyl quinuclidine et 1,02 g de chlorhydrate de 0-2-propynylhydroxylamine dans 30 cm3 de méthanol. On évapore à sec, chromatographie le résidu sur silice (éluant : méthanol-éther éthylique). On ajoute une solution aqueuse de carbonate de potassium, extrait à l'acétate d'éthyle, sèche et distille à 170¤C sous 0,2 mbar. Après refroidissement, on obtient 1,1 g de produit attendu. F= 56-58¤C.

| Analyse : $C_{12}H_{18}N_2O$ | | | |
|---|---|---|---|
| Calculé : | C% 69,87 | H% 8,80 | N% 13,58 |
| Trouvé : | 69,26 | 8,69 | 13,43 |

**Exemple 6 : Chlorhydrate de O-éthyloxime de (1-azabicyclo [2,2,2] octan-3-yl) éthanone.**

On chauffe 20 minutes au reflux 1,9 g de chlorhydrate de 3-acétyl quinuclidine et 0,98 g de chlorhydrate de O-éthylhydroxylamine dans 30 cm3 de méthanol. On concentre à sec, purifie par chromatographie sur alumine (éluant : chlorure de méthylène-méthanol 9-1). On acidifie le milieu réactionnel à l'aide d'acide chlorhydrique gazeux, puis évapore à sec. On cristallise dans l'éthanol, filtre, rince à l'éther et recueille 1,3 g de produit attendu. F = 198-200¤C.

| Analyse : $C_{11}H_{21}N_2O$ | | | |
|---|---|---|---|
| Calculé : | C% 56,76 | H% 9,10 | N% 12,04 |
| Trouvé : | 55,85 | 9,02 | 11,92 |

**Exemple 7 : Oxime de (1-azabicyclo [2,2,2] octan-3-yl) éthanone et son chlorhydrate.**

On chauffe au reflux 2,2 g de chlorhydrate de 3-acétyl quinuclidine et 0,81 g de chlorhydrate d'hydroxylamine dans 30 cm3 de méthanol. On concentre à sec, purifie par chromatographie sur alumine (éluant : chlorure de méthylène-méthanol 9-1), recueille 0,2 g de base, acidifie à l'aide d'acide chlorhydrique gazeux, évapore à sec. On cristallise dans l'éthanol, filtre, rince à l'éther et recueille 1,45 g de produit attendu sous forme de chlorhydrate.

| Analyse effectuée sur la base : $C_9H_6N_2O$ | | | |
|---|---|---|---|
| Calculé : | C% 64,25 | H% 9,59 | N% 16,65 |
| Trouvé : | 64,33 | 9,61 | 16,64 |

Point de fusion de la base F = 96-98¤C.

En opérant comme indiqué ci-dessus à partir des composés appropriés, on a préparé les produits suivants :

**Exemple 8 : Chlorhydrate de l'oxime de (1-azabicyclo [2,2,2] octan-3-carboxaldéhyde.**

**Exemple 9 : Oxime de (1-azabicyclo [2,2,2] octan-4-carboxaldéhyde.**

**Exemple 10 : Chlorhydrate de O-méthyloxime de (1-azabicyclo [2,2,2] octan-4-carboxal-déhyde.**

On refroidit à -50¤/-60¤C 1,09 cm3 de chlorure d'oxalyle dans 30 cm3 de dichlorométhane et ajoute goutte à goutte 1,6 cm3 de diméthylsulfoxyde et agite pendant 15 minutes. On ajoute 1,6 g de 4-hydroxyméthyl quinuclidine préparé comme indiqué dans Helv. Chimica Acta 57 (8) 2332 (1974) dans 50 cm3 environ de dichlorométhane et on agite pendant 2 heures la suspension à -50¤/-60¤C. On ajoute goutte à goutte 7,89 cm3 de triéthylamine, laisse revenir à température ambiante, ajoute 0,95 g de chlorhydrate de O-méthyl hydroxylamine dissous dans 30 cm3 de méthanol et laisse à température ambiante pendant 16 heures. On évapore les solvants, reprend au dichlorométhane, alcalinise avec de la soude 2N. On sépare les 2 phases, extrait la phase aqueuse avec du dichlorométhane, sèche et évapore. On dissout le résidu dans le diéthyléther et transforme en chlorhydrate par un courant d'acide chlorhydrique, on filtre le solide, le cristallise dans le mélange éthanol-diéthyléther et obtient 0,91 g de solide blanc qui se décompose à 271-271,5¤C.

| Analyse : | | | |
|---|---|---|---|
| Calculé : | C% 52,81 | H% 8,37 | N% 13,69 |
| Trouvé : | 52,90 | 8,36 | 13,55 |

**Exemple 11 : Oxalate de l'O-propynyloxime de 1-azabicyclo [2,2,2] octan-4-carboxaldéhyde.**

En opérant de manière analogue à celle décrite à l'exemple 10 et en employant la O-propargyl hydroxylamine, on a obtenu le produit attendu. F = 162¤C (décomposition).

**Exemple 12 : O-éthyloxime de 1-azabicyclo [2,2,2] octan-4-carboxaldéhyde.**

On opérant comme indiqué ci-dessus à partir des composés appropriés, on a obtenu le produit attendu.

**Exemple 13 : O-méthyloxime de 1-azabicyclo [2,2,2] octan-4-yl éthanone et sons chlorhydrate.**

On agite pendant 3 heures à température ambiante, une solution comprenant 0,65 g de 4-acétyl quinuclidine préparée comme indiqué dans Helv. Chimica Acta 55 (7) 2439 (1972) et 0,29 g de chlorhydrate de O-méthyl hydroxylamine dans 20 cm3 de méthanol. On évapore à sec, reprend le résidu dans de la soude 2M, extrait au chloroforme, sépare la phase organique, la sèche, évapore le solvant. On reprend le résidu dans l'éthanol, acidifie avec une solution éthanolique d'acide chlorhydrique à 20% et précipite à l'éther éthylique. On filtre et recristallise dans le mélange éthanol-éther pour obtenir 0,7 g de produit attendu. F > 300¤C.

| Analyse : $C_{10}H_{18}N_2O$ : 218,728 | | | |
|---|---|---|---|
| Calculé :<br>Trouvé : | C% 54,91<br>54,87 | H% 8,76<br>8,70 | N% 12,81<br>12,53 |

**Exemple 14 : O-propynyloxime de 1-azabicyclo [2,2,2] octan-4-yl éthanone.**

En opérant comme à l'exemple 13 à partir des composés appropriés, on a obtenu le produit attendu.

**Exemple 15 : O-méthyloxime de 1-azabicyclo [2,2,1] heptan-3-carboxaldéhyde et son sesqui oxalate.**

**Stade A :** 3-méthoxyméthylidène 1-azabicyclo [2,2,1] heptane.

A une suspension de 3,86 g de chlorure de (méthoxyméthyl) triphényl phosphonium dans 20 cm3 d'éther anhydre, on ajoute lentement sous pression réduite et sous agitation, 7 cm3 d'une solution 1,6M de n-Buli. On agite le mélange réactionnel 15 minutes à température ambiante, refroidit à -35¤C et ajoute goutte à goutte 1,25 g de 1-azabicyclo [2,2,1] heptan-3-one, décrit dans J. Org. Chem. 1969, 34, 3674, dans l'éther en maintenant la température -35¤C. On laisse le mélange réactionnel revenir à température ambiante, laisse sous agitation et sous atmosphère d'azote pendant 16 heures. Le mélange réactionnel est filtré pour éliminer le chlorure de lithium et l'oxyde de triphénylphosphine. Après évaporation de l'éther, le résidu est distillé sous pression réduite. On recueille le produit à environ 100¤C/15 mm Hg. On obtient 0,50 g de produit (liquide incolore).

**Stade B :** 1-azabicyclo [2,2,1] heptan-3-carboxaldéhyde O-méthyloxime et son sesqui oxalate.

A une solution de 0,5 g de produit obtenu au stade A dans 5 cm3 de chloroforme, on ajoute en 10 minutes, 1 cm3 d'acide perchlorique à 70%. On agite 2 heures à température ambiante, ajoute 0,3 g de chlorhydrate de O-méthyl hydroxylamine dissous dans 1 cm3 d'eau et 3 cm3 de méthanol. On agite à température ambiante pendant 24 heures, concentre sous pression réduite, alcalinise avec de la soude 2N et extrait à l'acétate d'éthyle. On sépare la phase organique, la sèche et évapore le solvant. On reprend le résidu huileux dans l'acétate d'éthyle et traite avec une quantité équimoléculaire d'acide oxalique dihydraté dissous dans le même solvant. On recristallise l'oxalate dans l'isopropanol et obtient 0,4 g de produit

attendu. F = 95-97¤C.

| Analyse : $C_8H_{14}N_2O$, 1,5 $C_2H_2O_4$ = $C_{11}H_{17}N_2O_7$. P.M. 289,267 | | | |
|---|---|---|---|
| Calculé :<br>Trouvé : | C% 45,67<br>45,95 | H% 5,92<br>5,98 | N% 9,68<br>9,85 |

On a préparé également le produit suivant :

**Exemple 16 : O-méthyloxime de (1-azabicyclo [2,2,1] heptan-3-yl) éthanone.**

**Exemples de compositions pharmaceutiques.**

**Exemple 17:**

On a préparé des comprimés répondant à la formule suivante :

| | |
|---|---|
| - Produit de l'exemple 1 ...<br>- Excipient q.s. pour un comprimé terminé à ... | 50 mg<br>300 mg |
| (Détail de l'excipient : lactose, talc, amidon de blé, amidon traité, amidon de riz, stéarate de magnésium). | |

**Exemple 18 :**

On a préparé des gélules répondant à la formule suivante :

| | |
|---|---|
| - Produit de l'exemple 1 ...<br>- Excipient q.s. pour une gélule terminée à ... | 60 mg<br>300 mg |
| (Détail de l'excipient : aérosil, talc, stéarate de magnésium). | |

**ETUDE PHARMACOLOGIQUE**

Toxicité aigüe.

L'essai est réalisé sur des souris mâles ($CD_1$ Charles Rivers) de 22 à 24 g, à jeun depuis 16 heures. On administre les produits par voie orale à la dose de 1000, 500, 250, 125, 62, 31 et 16 mg/kg. On note la mortalité pendant les 7 jours suivant le traitement.

| Exemple | $DL_{50}$ mg/kg |
|---|---|
| 2 | 150 |
| 4 | 100 |
| Acéclidine,HCl | 250 |

Test de l'iléon isolé de cobaye.

On prélève les fragments d'iléon de cobayes tués par décapitation. L'iléon isolé est placé dans 10 cm3 de solution de Tyrode à 37¤C et aéré par un mélange d'oxygène (95%) et de gaz carbonique (5%). Les contractions dues aux produits sont enregistrées à l'aide d'un capteur relié à un polygraphe. Les produits à tester sont ajoutés, aux concentrations comprises entre $1.10^{-3}$M et $1.10^{-8}$M/l.

Les produits présentant un effet contracturant sont testés vis-à-vis de l'atropine et de l'hexaméthonium pour établir si l'activité est de type "muscarinique" ou "nicotinique".

L'activité agoniste est exprimée en $pD_2$ (logarithme négatif de la dose qui produit 50% de l'effet maximum).

| Exemple | $pD_2$ |
|---|---|
| 1 | 7,30 |
| 4 | 7,02 |
| 15 | 7,63 |
| Acéclidine,HCl | 6,98 |

Activité diarrhéique.

Le test est réalisé sur des souris mâles ($CD_1$ Charles Rivers) pesant 25 à 30 g, à jeun depuis 6 heures. Le produit dissous à 5% dans du méthocel est administré par voie orale, au moyen d'une sonde oesophagienne.

Des animaux témoins ne reçoivent que l'excipient (20 ml/kg).

Après traitement, les animaux sont mis séparément dans des cages dont le fond est recouvert de papier buvard et sont mis en observation pendant 30, 60, 120 et 180 minutes.

Les feuilles de papier absorbant sont changées après chaque observation.

La consistance des fèces est évaluée selon la méthode de Randall et Baruth (Arch. Int. Pharmacodyn. 220, 94, 1976) en suivant l'échelle des valeurs suivantes.

0 : consistance ferme,
1 : fèces légèrement molles avec ou sans auréole humide,
2 : fèces légèrement molles avec présence d'un cercle humide bien défini,
3 : fèces molles avec présence d'un grand cercle humide,
4 : fèces sans consistance avec présence d'un très grand cercle humide.

Pour chaque produit, on a noté la dose qui provoque une diarrhée chez 50% des animaux selon la méthode de Miller et Tainter (Proc. Soc. Exp. Biol. Med., 57 , 261, 1944).

| Exemple | $DE_{50}$ mg/kg |
|---|---|
| 1 | 1,1 |
| 4 | 3,0 |
| 15 | 0,065 |
| Acéclidine,HCl | 4,0 |

Activité hypothermique.

Le test est réalisé sur des souris mâles ($CD_1$ Charles Rivers) pesant 25 à 30 g, à jeun depuis 6 heures.

La température du corps est notée au moyen d'un thermocouple placé dans le rectum à environ 1,5 cm et relié à un enregistreur de température électrique.

Les produits sont administrés par voie orale ou sous-cutanée et les températures sont notées à l'instant 0 puis 30 minutes, 1 heure, 2 heures et 2 heures et demie après traitement.

On évalue le degré d'hypothermie comme la différence entre les animaux traités et les témoins et on détermine la dose nécessaire pour réduire de 1¤C la température du corps.

| Exemple | Dose efficace (-1¤C) en mg/kg | |
|---|---|---|
| | V.O | V.SC |
| 1 | 0,19 | 0,18 |
| 4 | 0,33 | 0,33 |
| 15 | 0,022 | 0,021 |
| Acéclidine,HCl | 6,4 | 2,1 |

On détermine la durée d'action des produits en utilisant des doses capables de réduire la température de 1¤ à 1,2¤C.

| Exemple | Dose mg/kg | administration | temps en minute de traitement | | | | |
|---|---|---|---|---|---|---|---|
| | | | 0 | 30 | 60 | 120 | 180 |
| 1 | 0,25 | os | ± 0 | -1,1 ** | -1,0 ** | ± 0 | ± 0 |
| | 0,25 | sc | ± 0 | -1,2 ** | -1,1 ** | ± 0 | ± 0 |
| 4 | 0,31 | os | ± 0 | -0,9 ** | -0,5 ** | - 0,1 | ± 0 |
| | 0,31 | sc | ± 0 | -0,9 ** | -0,6 ** | - 0,1 | - 0,1 |
| 15 | 0,025 | os | ± 0 | -1,2 ** | -1,1 ** | - 0,1 | + 0,1 |
| | 0,à25 | sc | - 0,1 | -1,3 ** | -1,2 ** | - 0,1 | ± 0 |
| Acéclidine,HCl | 7,50 | os | ± 0 | -1,1 ** | -0,3 * | ± 0 | + 0,1 |
| | 2,50 | sc | ± 0 | -1,0 ** | -0,1 | + 0,1 | + 0,1 |

** Valeurs différentes des témoins d'une manière significative (p< 0,01)
* Valeurs différentes des témoins d'une manière significative (p< 0,05).

**Revendications**

1.- Les composés de formule générale (I) :

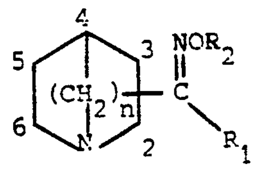

(I)

dans laquelle

- n représente le nombre 1, 2 ou 3,

- $R_1$ et $R_2$, identiques ou différents, représentent un atome d'hydrogène, un radical alkyl linéaire ramifié ou cyclique, un radical alkényle ou un radical alkynyle pouvant renfermer jusqu'à 6 atomes de carbone, $R_1$ et $R_2$ pouvant éventuellement être substitués par un radical hydroxy, un radical alkoxy renfermant jusqu'à 6 atomes de carbone, un radical aralkyle renfermant jusqu'à 14 atomes de carbone, un radical $COOalc_1$ dans lequel $alc_1$ représente un radical alkyle renfermant jusqu'à 6 atomes de carbone, un radical $-CON(alc_2)_2$ dans lequel $alc_2$ représente un radical alkyle renfermant jusqu'à 6 atomes de carbone, le radical $-C(R_1)=NOR_2$ étant en position 2, 3 ou 4, sous toutes les formes isomères possibles et leurs mélanges, ainsi que leurs sels d'addition avec les acides organiques ou minéraux.

2.- Les composés de formule (I) tels que définis à la revendication 1, dans lesquels $R_2$ représente un radical alkyle, alkényle ou alkynyle renfermant jusqu'à 6 atomes de carbone ainsi que leurs sels d'addition avec les acides organiques ou minéraux.

3.- Les composés de formule (I) tels que définis à la revendication 1 ou 2, dans lesquels le radical $-C(R_1)=NOR_2$ est en position 3, ainsi que leurs sels d'addition avec les acides organiques ou minéraux.

4.- Les composés de formule (I) tels que définis à l'une quelconque des revendications 1 à 3, dans lesquels n représente le nombre 1, ainsi que leurs sels d'addition avec les acides organiques ou minéraux.

5.- Les composés de formule (I) tels que définis à l'une quelconque des revendications 1 à 3, dans lesquels n représente le nombre 2, ainsi que leurs sels d'addition avec les acides organiques ou minéraux.

6.- Les composés de formule (I) tels que définis à l'une quelconque des revendications 1 à 5, dans lesquels $R_1$ représente un atome d'hydrogène ainsi que leurs sels d'addition avec les acides organiques ou minéraux.

7.- Les composés de formule (I) tels que définis à l'une quelconque des revendications 1 à 5, dans lesquels $R_1$ représente un radical méthyle ainsi que leurs sels d'addition avec les acides organiques ou minéraux.

8.- Les composés de formule (I) tels que définis à l'une quelconque des revendications 1 à 3 et 5 à 7 dans lesquels n = 2, $R_1$ et $R_2$ ne représentent pas chacun un atome d'hydrogène.

9.- Les composés de formule (I) tels que définis à l'une quelconque des revendications 1 à 8, dans lesquels $R_2$ représente un radical alkyle linéaire, ramifié ou cyclique renfermant jusqu'à 4 atomes de carbone ainsi que leurs sels d'addition avec les acides organiques ou minéraux.

10.- Les composés de formule (I) tels que définis à l'une quelconque des revendications 1 à 9 dans lesquels $R_2$ représente un radical méthyle, ainsi que leurs sels d'addition avec les acides organiques ou minéraux.

11.- Les composés de formule (I) définis à la revendication 1, dont les noms suivent :

- le O-méthyloxime de 1-azabicyclo [2,2,2] octan-3-carboxaldéhyde ainsi que ses sels d'addition avec les acides organiques ou minéraux,

- le O-méthyloxime de 1-azabicyclo [2,2,2] octan-3-yl éthanone ainsi que ses sels d'addition avec les acides organiques ou minéraux et particulièrement son chlorhydrate

- le O-méthyloxime de 1-aza-bicyclo [2,2,1] heptan-3-carboxaldéhyde ainsi que ses sels d'addition avec les acides organiques ou minéraux et particulièrement son sesqui oxalate.

12.- A titre de médicaments, les composés de formule (I) tels que définis à l'une quelconque des revendications 1 à 10, ainsi que leurs sels d'addition avec les acides organiques ou minéraux.

13.- A titre de médicament, les composés définis à la revendication 11.

14.- Les compositions pharmaceutiques renfermant comme principe actif au moins un médicament défini à la revendication 12 ou 13.

15.- Procédé de préparation des composés de formule (I) tels que définis à l'une quelconque des revendications 1 à 11, caractérisé en ce que l'on soumet un composé de formule générale (II) :

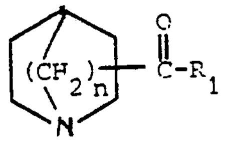

(II)

dans laquelle n et $R_1$ conservent la même signification que précédemment, à l'action d'un composé de formule générale (III) :

$R_2ONH_2$ (III)

dans laquelle $R_2$ conserve la même signification que précédemment pour obtenir le composé de formule (I) correspondant, que l'on salifie le cas échéant.

Revendications pour les Etats contractants suivant: ES, GR

1.- Procédé pour préparer les composés de formule générale (I) :

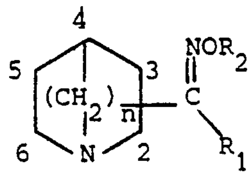

dans laquelle

- n représente le nombre 1, 2 ou 3,
- $R_1$ et $R_2$, identiques ou différents, représentent un atome d'hydrogène, un radical alkyle linéaire ramifié ou cyclique, un radical alkényle ou un radical alkynyle pouvant renfermer jusqu'à 6 atomes de carbone, $R_1$ et $R_2$ pouvant éventuellement être substitués par un radical hydroxy, un radical alkoxy renfermant jusqu'à 6 atomes de carbone, un radical aralkyle renfermant jusqu'à 14 atomes de carbone, un radical $COOalc_1$ dans lequel $alc_1$ représente un radical alkyle renfermant jusqu'à 6 atomes de carbone, un radical $-CON(alc_2)_2$ dans lequel $alc_2$ représente un radical alkyle renfermant jusqu'à 6 atomes de carbone, le radical $-C(R_1)-=NOR_2$ étant en position 2, 3 ou 4, sous toutes les formes isomères possibles et leurs mélanges, ainsi que leurs sels d'addition avec les acides organiques ou minéraux, caractérisé en ce que l'on soumet un composé de formule générale (II) :

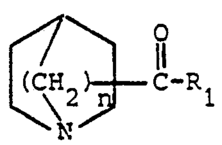

(II)

dans laquelle n et $R_1$ conservent la même signification que précédemment, à l'action d'un composé de formule générale (III) :

$R_2ONH_2$ (III)

dans laquelle $R_2$ conserve la même signification que précédemment, pour obtenir le composé de formule (I) correspondant, que l'on salifie le cas échéant.

2.- Procédé selon la revendication 1, caractérisé en ce que l'on utilise au départ un produit de formule (III) dans laquelle $R_2$ représente un radical alkyle, alkényle ou alkynyle renfermant jusqu'à 6 atomes de carbone.

3.- Procédé selon la revendication 1 ou 2, caractérisé en ce que l'on utilise au départ un produit de formule (II) dans laquelle le radical

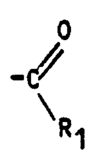

est en position 3.

4.- Procédé selon l'une quelconque des revendications 1 à 3, caractérisé en ce que l'on utilise au départ un produit de formule (II) dans laquelle n représente le nombre 1.

5.- Procédé selon l'une quelconque des revendications 1 à 3, caractérisé en ce que l'on utilise au départ un produit de formule (II) dans laquelle n représente le nombre 2.

6.- Procédé selon l'une quelconque des revendications 1 à 5, caractérisé en ce que l'on utilise au départ un produit de formule (II) dans laquelle $R_1$ représente un atome d'hydrogène.

7.- Procédé selon l'une quelconque des revendications 1 à 5, caractérisé en ce que l'on utilise au départ un produit de formule (II) dans laquelle $R_1$ représente un radical méthyle.

8.- Procédé selon l'une quelconque des revendications 1 à 3 et 5 à 7, caractérisé en ce que l'on utilise au départ un produit de formule (II) et un produit de formule (III) tels que lorsque n = 1, $R_1$ et $R_2$ ne représentent pas chacun un atome d'hydrogène.

9.- Procédé selon l'une quelconque des revendications 1 à 8, caractérisé en ce que l'on utilise au départ un produit de formule (III) dans laquelle $R_2$ représente un radical alkyle linéaire, ramifié ou cyclique, renfermant jusqu'à 4 atomes de carbone.

10.- Procédé selon l'une quelconque des revendications 1 à 9, caractérisé en ce que l'on utilise au départ un produit de formule (III) dans laquelle $R_2$ représente un radical méthyle.

11.- Procédé selon l'une quelconque des revendications 1 à 10, caractérisé en ce que l'on utilise au départ des produits de formule (II) de formule (III) tels que l'on prépare l'un des composés dont les noms suivent : - le O-méthyloxime de 1-azabicyclo [2,2,2] octan-3-carboxaldéhyde ainsi que ses sels d'addition avec les acides organiques ou minéraux,

- le O-méthyloxime de 1-azabicyclo [2,2,2] octan-3-yl éthanone ainsi que ses sels d'addition avec les acides organiques ou minéraux et particulièrement son chlorhydrate

- le O-méthyloxime de 1-aza-bicyclo [2,2,1] heptan-3-carboxaldéhyde ainsi que ses sels d'addition avec les acides organiques ou minéraux et particulièrement son sesqui oxalate.